# FASCICULE DE BREVET EUROPEEN

(11) **EP 0 387 136 B1**
(45) Date de publication et mention de la délivrance du brevet: **27.07.1994**
(21) Numéro de dépôt: 90400594.9
(22) Date de dépôt: 05.03.1990
(51) Int. Cl.: A61L 2/06

(54) **Dispositif d'injection de liquide le long des parois intérieures d'un autoclave**
Vorrichtung zum Einspritzen von Flüssigkeiten entlang der Innenwände eines Autoklaven
Liquid-injection device along the inner walls of an autoclave

(30) Priorité: 09.03.1989 FR 8903088
(43) Date de publication de la demande: 12.09.1990
(73) Titulaire: CENTRE REGIONAL DE TRANSFUSION SANGUINE DE LILLE, 59012 Lille (FR); SOCIETE D'UTILISATION SCIENTIFIQUE ET INDUSTRIELLE DU FROID USIFROID, F-78130 Maurepas (FR)
(72) Inventeur: Sierakowski Dominique, F-59116 Houplines (FR); Baron Michel, F-78370 Plaisir (FR)
(74) Mandataire: Lhuillier, René

(56) Documents cités:
- Eléments de la technique relevés: néant.

## Description

L'invention qui se rapporte aux autoclaves, concerne plus précisément un dispositif d'injection de liquide, tel que de l'eau, le long des parois intérieures d'un autoclave pour assurer son ruissellement sur lesdites parois.

L'utilisation d'autoclaves pour assurer la stérilisation de produits ou d'objets divers est très largement répandue, que ce soit dans l'industrie agro-alimentaire ou dans les laboratoires pharmaceutiques ou autres. On sait que la chambre interne d'un autoclave, remplie des produits ou objets à stériliser, est soumise à une température et une pression bien déterminées, sous atmosphère d'eau ou de vapeur d'eau. Pour ne pas dépasser à l'intérieur de cette enceinte fermée une température qui pourrait être préjudiciable à la qualité du produit à stériliser, ni une pression qui pourrait occasionner des dégâts aux boîtes ou récipients contenant ces produits, il est habituel d'injecter de la vapeur dans l'autoclave à une température nettement supérieure à la température de stérilisation, puis d'abaisser la température dans l'autoclave jusqu'à cette température de stérilisation sans modifier la pression, puis enfin de refroidir réellement l'intérieur de l'autoclave. Avantageusement, l'abaissement de température est assuré en introduisant de l'air et de l'eau, l'eau étant introduite par pulvérisation pour ne pas entrer en contact direct avec les récipients ou produits à stériliser. Ces opérations nécessitent par conséquent d'équiper l'autoclave de rampes d'injection de vapeur et de rampes de pulvérisation d'eau, distribuées par des vannes appropriées. Il est connu également de recueillir au fond de la cuve les condensats qui se sont accumulés, et de les évacuer vers l'égoût, ou encore de les recycler dans la cuve grâce à une pompe. Dans ce dernier cas, ils sont refroidis par un injecteur à plaques, puis réinjectés par une pompe appropriée ou par les rampes de pulvérisation d'eau elles-mêmes auxquelles ils sont raccordés.

Bien que ces solutions classiques soient largement utilisées et donnent satisfaction, elles ne résolvent pas cependant tous les problèmes qui peuvent subsister et notamment ceux liés aux difficultés de refroidissement de la cuve et aussi à la stérilisation des condensats.

Il est apparu à la demanderesse que le recyclage de ces condensats, et l'injection d'eau déminéralisée à condition qu'ils soient assurés de façon particulière, en ruisselant le long des parois intérieures de l'autoclave, permettraient de résoudre avantageusement ces problèmes.

Un objet de la présente invention consiste donc en un dispositif d'injection de liquide à l'intérieur d'un autoclave dont les condensats sont récuperés à la partie inférieure et réinjectés à la partie supérieure en même temps que de l'eau déminéralisée, par un conduit comportant une pompe et un échangeur de refroidissement, dispositif selon lequel au moins un conduit assurant l'injection de ce liquide débouche à l'intérieur de l'autoclave au-dessus d'un godet longitudinal horizontal s'étendant sur la paroi intérieure de la cuve d'autoclave lequel godet présentant un profil particulier lui permettant de distribuer le liquide sur toute la longueur de la cuve pour qu'il s'échappe par une fente profilée s'étendant sur toute la longueur du godet à proximité de la paroi interne de la cuve et ruissèle le long de ladite paroi.

Plus précisément, le godet a une section sensiblement rectangulaire dont un de ses côtés sensiblement vertical sert à le fixer à la paroi intérieure de la cuve tandis que sa base présente une faible inclinaison vers le bas en direction de ladite paroi, et l'extrémité de la base du côté de la paroi de la cuve est profilée en forme d'une pointe orientée vers le bas, dont la face tournée vers la cuve est sensiblement parallèle à celle-ci.

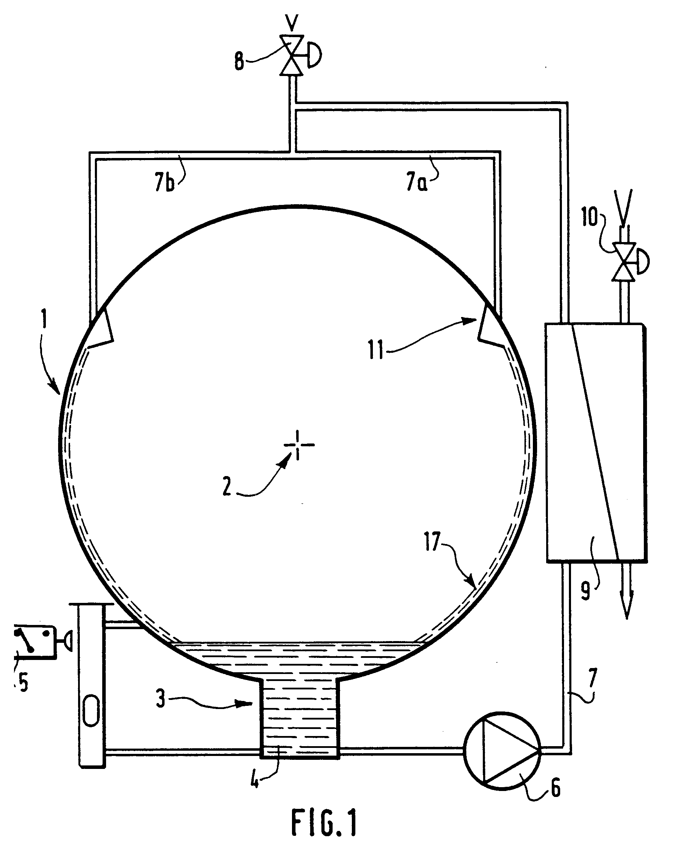

D'autres caractéristiques particulières et avantages de l'invention ressortiront de la description qui va suivre d'une forme de réalisation qui doit être considérée à titre d'exemple et qui fait référence aux dessins annexés qui représentent :
- Figure 1 une vue schématique en coupe d'un autoclave équipé du dispositif d'injection de liquide
- Figure 2 une vue en coupe à plus grande échelle du godet du dispositif d'injection
On voit à la figure 1 une cuve d'autoclave 1 d'axe longitudinal 2 dont le fond inférieur est pourvu d'une rigole 3 servant à recueillir un mélange 4 de condensats et d'eau déminéralisée. Un contacteur de niveau 5 surveille la hauteur de liquide dans le fond de la cuve en vue de l'ouverture de vannes d'évacuation de sécurité dès que le niveau dépasse un niveau autorisé prédéterminé.

Une pompe 6 permet de prélever le fluide à la partie inférieure de la rigole 3 et de le recycler par le conduit 7 vers le haut de la cuve. Le conduit 7 se divise dans cette zone haute en deux branches 7a et 7b qui débouchent dans la cuve 1 environ aux trois quarts de sa hauteur et symétriquement par rapport à son plan longitudinal. Le conduit 7, dans sa partie terminale reçoit l'appoint éventuel d'eau déminéralisée, distribuée par une vanne 8. Sur le conduit 7 est interposé un échangeur à plaque 9 alimenté en eau froide par la vanne 10, destiné à refroidir le fluide circulant dans le conduit 7.

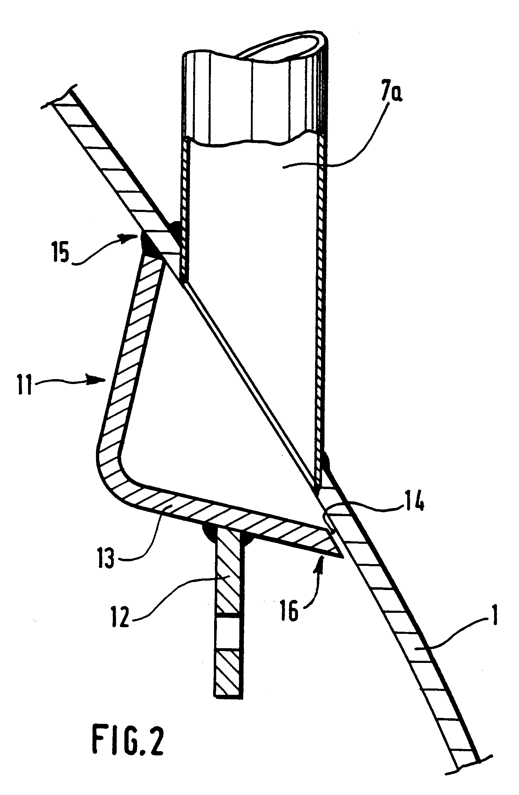

Les conduits 7a et 7b débouchent dans la cuve à l'aplomb d'un godet longitudinal horizontal 11 qui s'étend sur la paroi intérieure de la cuve 1, sur toute sa longueur et ses deux côtés. Ce godet présente un profil particulier, bien visible sur la figure 2. La bordure superieure du godet 11 est soudée, sur toute sa longueur à la paroi interne de la cuve par une soudure 15. La base 13 du godet, par contre, est soutenue au moins de place en place par une cornière 12 elle-même fixée à la cuve de façon non représentée. On notera que le godet a une section sensiblement rectangulaire et qu'il est fixé par un de ses côtés sensiblement vertical à la paroi interne de la cuve à un emplacement et selon une orientation tels que la base 13 présente une faible inclinaison vers le bas en direction de ladite paroi. La base 13 du godet ne vient pas en contact avec la paroi de la cuve dont elle est séparée par une petite fente 14 s'étendant sur toute la longueur du godet. L'extrémité de cette base est profilée en forme d'une pointe 16, orientée vers le bas, dont la face tournée vers la cuve est sensiblement parallèle à celle-ci.

Quand la pompe 6 est en fonctionnement elle prélève le liquide dans la rigole 3 et le recycle vers le haut par le conduit 7 en passant par l'échangeur 9 où il est refroidi. Ce liquide éventuellement additionné d'eau déminéralisée fournie par la vanne 8 est distribuée par les conduits 7a et 7b dans les godets 11 qui se remplissent sur toute leur longueur. Le liquide s'échappe par la fente 14 de façon laminaire telle qu'il ruissèle régulièrement le long de la cuve en formant un film de liquide 17 qui couvre donc les deux parois latérales de la cuve 1 jusqu'à la partie inférieure. Bien entendu, pour que ce film de liquide soit réparti sur toute la longueur de la cuve aussi uniformément que possible, il peut être judicieux d'alimenter les godets 11 en deux ou plusieurs points de leur longueur par des dérivations des conduits d'alimentation 7a et 7b.

Ce ruissellement permet la stérilisation des condensats si les parois de la cuve ont une température supérieure à 100°C et le refroidissement de la cuve si on alimente l'échangeur intermédiaire en eau froide.

## Revendications

1. Dispositif d'injection de liquide à l'intérieur d'un autoclave dont les condensats sont récupérés à la partie inférieure et réinjectés à la partie supérieure en même temps que de l'eau déminéralisée, par un conduit comportant une pompe et un échangeur de refroidissement, caracterisé en ce que au moins un conduit (7) assurant l'injection de ce liquide débouche à l'intérieur de l'autoclave au-dessus d'un godet longitudinal horizontal (11) s'étendant sur la paroi intérieure de la cuve d'autoclave (1), lequel godet présentant un profil particulier lui permettant de distribuer le liquide sur toute la longueur de la cuve pour qu'il s'échappe par une fente profilée (14) s'étendant sur toute la longueur du godet à proximité de la paroi interne de la cuve et ruissèle le long de ladite paroi.

2. Dispositif d'injection selon la revendication 1, caractérisé en ce que le godet (11) a une section sensiblement rectangulaire dont un de ses côtés sensiblement vertical sert à le fixer à la paroi intérieure de la cuve 1, tandis que sa base (13) présente une faible inclinaison vers le bas en direction de ladite paroi.

3. Dispositif d'injection selon les revendications 1 et 2, caractérisé en ce que l'extrémité de la base (13) du côté de la paroi de la cuve (1) est profilée en forme d'une pointe (16) orientée vers le bas, dont la face tournée vers la cuve est sensiblement parallèle à celle-ci.

## Claims

1. Device for injecting liquid into the interior of an autoclave, the condensates of which are recovered at the lower part and re-injected at the upper part at the same time as demineralised water, via a pipe comprising a pump and a cooling exchanger, characterised in that at least one pipe (7) for injecting that liquid opens out inside the autoclave above a horizontal longitudinal receptacle (11) extending along the internal wall of the autoclave tank (1), which receptacle has a particular profile allowing it to distribute the liquid along the entire length of the tank so that it escapes via a profiled slot (14) extending along the entire length of the receptacle near the internal wall of the tank and flows along the said wall.

2. Injection device according to claim 1, characterised in that the receptacle (11) has a substantially rectangular section, one of the sides of which, which is substantially vertical, serves to secure it to the internal wall of the tank (1), while its base (13) is slightly inclined towards the bottom in the direction towards the said wall.

3. Injection device according to claims 1 and 2, characterised in that the end of the base (13) next to the wall of the tank (1) is profiled in the shape of a point (16) that is oriented towards the bottom and the face of which that is turned towards the tank is substantially parallel therewith.

## Patentansprüche

1. Vorrichtung zum Einspritzen von Flüssigkeit ins Innere eines Autoklaven, dessen Kondensate am Unterteil wiedergewonnen und am Oberteil wiedereingespritzt werden, zur gleichen Zeit wie entmineralisiertes Wasser, durch eine Leitung, die eine Pumpe enthält und einen Kühler,
**dadurch gekennzeichnet,** daß wenigstens eine die Einspritzung dieser Flüssigkeit sicherstellende Leitung (7) im Inneren des Autoklaven mündet, über einem horizontalen Längsnapf (11), der sich auf der Innenwand des Autoklavenbehälters (1) erstreckt, wobei dieser Napf ein besonderes Profil aufweist, das ihm ermöglicht, die Flüssigkeit über die gesamte Länge des Behälters zu verteilen, so daß sie durch einen profilierten Schlitz (14) entweicht, der sich über die ganze Länge des Behälters erstreckt, nahe der Innenwand des Behälters, und an der genannten Wand entlang herabrinnt.

2. Einspritzvorrichtung nach Anspruch 1, dadurch gekennzeichnet, daß der Napf (11) einen im wesentlichen recktwinkligen Querschnitt hat, wobei die im wesentlichen vertikale seiner Seiten dazu dient, ihn an der Innenwand des Behälters (1) zu befestigen, während seine Basis (13) in Richtung der genannten Wand eine schwache Neigung aufweist.

3. Einspritzvorrichtung nach einem der Ansprüche 1 und 2, dadurch gekennzeichnet, daß das Ende der Basis (13) auf der Seite der Wand des Behälters (1) die Form einer Spitze (16) aufweist, nach unten gerichtet, deren dem Behälter zugekehrte Fläche im wesentlichen parallel zu diesem ist.
